(19) Europäisches Patentamt
European Patent Office
Office européen des brevets

(11) Numéro de publication: **0 500 413 B1**

(12) # FASCICULE DE BREVET EUROPEEN

(45) Date de publication du fascicule du brevet: **20.09.95**

(51) Int. Cl.[6]: **B01J 29/04**, C10G 35/06, C07C 5/41, C07C 2/00

(21) Numéro de dépôt: **92400333.8**

(22) Date de dépôt: **07.02.92**

Le dossier contient des informations techniques présentées postérieurement au dépôt de la demande et ne figurant pas dans le présent fascicule.

(54) **Utilisation d'un catalyseur de type galloaluminosilicate en aromatisation des hydrocarbures contenant entre 2 et 7 atomes de carbone par molecule.**

(30) Priorité: **19.02.91 FR 9102066**
**19.02.91 FR 9102067**

(43) Date de publication de la demande:
**26.08.92 Bulletin 92/35**

(45) Mention de la délivrance du brevet:
**20.09.95 Bulletin 95/38**

(84) Etats contractants désignés:
**BE DE GB IT NL**

(56) Documents cités:
**EP-A- 517 891**
**EP-A- 0 351 312**
**EP-A- 0 469 951**
**EP-A- 0 342 075**
**EP-A- 0 400 987**
**FR-A- 2 653 764**

(73) Titulaire: **INSTITUT FRANCAIS DU PETROLE**
**4, Avenue de Bois Préau**
**F-92502 Rueil-Malmaison (FR)**

(72) Inventeur: **Alario, Fabio**
**70 bis, rue Georges Clémenceau**
**F-94210 La Varenne (FR)**
Inventeur: **Berthelin, Maurice**
**1, avenue du Dauphiné,**
**Serezin du Rhone**
**F-69360 Saint Symphorin d'Ozon (FR)**
Inventeur: **Brunard, Nathalie**
**5, quai des Etroits**
**F-69005 Lyon (FR)**
Inventeur: **Joly, Jean-François**
**20, rue Béranger**
**F-75003 Paris (FR)**
Inventeur: **Kolenda, Frédéric**
**7, rue Abbé Papon**
**F-69005 Lyon (FR)**
Inventeur: **Thery, Michel**
**181 A, route des Condamines Charly**
**F-69390 Vernaison (FR)**

**EP 0 500 413 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne :

- l'utilisation d'un catalyseur comprenant une zéolithe de structure MFI, synthétisée en milieu fluorure en l'absence d'agents organiques, contenant du silicium et de l'aluminium et/ou du gallium dans les réactions d'aromatisation des gaz légers contenant 2 à 7 atomes de carbone par molécule en présence ou non d'oléfines.

La zéolithe MFI, sous forme hydrogène, utilisée dans la présente invention est issue de la zéolithe MFI décrite et revendiquée dans la demande de brevet EP-A-517891, et a :

(a) La formule générale approchée suivante :

$$M^{+}_{(x+y)}(Si_{(96-(x+y))}Al_xGa_yO_{192})^{(x+y)-}$$

où M représente un cation alcalin et/ou un cation ammonium et/ou un proton, x est un nombre compris entre 0 et 24, y est un nombre compris entre 0 et 24, (x + y) est un nombre compris entre 0,64 et 24 et de préférence entre 1,88 et 19,2.

(b) Un diagramme de diffraction des rayons X de sa forme ammonium représenté dans le tableau 1 de la demande de brevet EP-A-517 891, ledit tableau 1 étant repris ci-après, et

(c) Une teneur en fluor de sa forme ammonium comprise entre 0,01 et 2,0 % en poids, de préférence entre 0,02 et 1 % en poids. Ladite zéolithe ayant été en outre synthétisée en milieu fluorure et en absence de composés organiques.

TABLEAU 1

| $D_{hkl}(10^{-10}m)$ | $I/I_o$ |
|---|---|
| 11,14 | 66 |
| 9,99 | 43 |
| 9,72 | 18 |
| 7,44 | 7 |
| 6,71 | 11 |
| 6,37 | 11 |
| 5,99 | 17 |
| 5,70 | 13 |
| 5,58 | 17 |
| 5,38 | 6 |
| 5,14 | 6 |
| 4,98 | 11 |
| 4,62 | 9 |
| 4,37 | 16 |
| 4,27 | 13 |
| 4,10 | 11 |
| 4,01 | 14 |
| 3,86 | 100 |
| 3,82 | 94 |
| 3,75 | 62 |
| 3,73 | 66 |
| 3,65 | 33 |
| 3,60 | 10 |
| 3,49 | 13 |
| 3,45 | 14 |
| 3,36 | 13 |
| 3,32 | 17 |
| 3,05 | 16 |
| 2,98 | 21 |
| 2,95 | 10 |

L'identification des zéolithes de type MFI suivant l'invention, décrite dans la demande de brevet EP-A-517 891, peut se faire de manière aisée à partir du diagramme de diffraction des rayons X de leur forme ammonium. Ce diagramme de diffraction peut être obtenu à l'aide d'un diffractomètre en utilisant la méthode classique des poudres avec le rayonnement $K_\alpha$ du cuivre. Un étalon interne permet de déterminer précisément les valeurs des angles 2 $\theta$ associées aux pics de diffraction. Les différentes distances interréticulaires $d_{hkl}$, caractéristiques de l'échantillon, sont calculées à partir de la relation de Bragg. L'estimation de l'erreur de mesure $\Delta$ ($d_{hkl}$) sur $d_{hkl}$ se calcule en fonction de l'erreur absolue $\Delta$ (2 $\theta$) affectée à la mesure de 2 $\theta$ par la relation de Bragg. En présence d'un étalon interne, cette erreur est minimisée et prise couramment égale à ± 0.05°. L'intensité relative I/Io affectée à chaque valeur de $d_{hkl}$ est estimée à partir de la hauteur du pic de diffraction correspondant.

Le tableau 1 représente le diagramme de diffraction des rayons X caractéristique de la forme ammonium de la zéolithe de type MFI selon la demande de brevet EP-A-517 891. Dans la colonne des $d_{hkl}$ on a représenté les valeurs extrêmes que peuvent prendre les différentes distances interréticulaires $d_{hkl}$. Chacune de ces valeurs doit être affectée de l'erreur de mesure généralement comprise entre ± 0,07 et 0,02 suivant la valeur de 2 $\theta$ ($d_{hkl}$ est exprimée en $10^{-10}$m).

La zéolithe MFI selon la demande de brevet EP-A-517 891 est caractérisée en ce qu'elle est synthétisée en l'absence de structurant organique. En effet, la synthèse de zéolithe MFI en milieu fluorure est connue de l'art antérieur de la demande de brevet EP-A-517 891, en ce qu'un agent organique est présent dans le milieu réactionnel de synthèse. Aussi l'art antérieur de la présente invention comprend le document EP-A-351 312 qui décrit un catalyseur d'aromatisation de gaz légers $C_2$-$C_4$ comprenant du gallium, une matrice, et une zéolithe MFI de type aluminosilicate ayant été synthétisée en milieu fluorure dont le milieu de synthèse comprend un agent organique. De même le document EP-A-469 951 décrit un catalyseur d'aromatisation de gaz légers $C_2$-$C_4$ comprenant une matrice et une zéolithe MFI de type galloalumino silicate ayant été synthétisée en milieu fluorure et dont le milieu de synthèse comprend un agent organique.

La zéolithe MFI forme hydrogène selon l'invention, synthétisée en milieu fluorure, a été préparée selon le procédé décrit et revendiqué dans la demande de brevet EP-A-517 891, procédé dont une description partielle est reprise ci-dessous à titre de référence :

(a) on forme un mélange réactionnel en solution ayant un pH inférieur à environ 9 et comprenant de l'eau, au moins une source de silice, au moins une source d'aluminium, au moins une source de gallium, au moins une source d'agent mobilisateur contenant des ions fluorures ($F^-$), au moins une source de cations alcalins (notés $A^+$), lesdits cations alcalins étant de préférence des cations sodium ($Na^+$), éventuellement au moins une source de cations ammonium ($NH_4^+$), ledit mélange réactionnel ayant une composition, en termes de rapport molaire, comprise dans les intervalles de valeurs suivants :

| | | |
|---|---|---|
| Si/(Al + Ga) | 7 - 60, | de préférence 14 - 50, |
| $F^-$/Si | 0,1 - 10, | de préférence 0,1 - 8, |
| $H_2O$/Si | 5 - 25, | de préférence 10 - 25, |
| $A^+$/Si | 0,1 - 10, | de préférence 0,1 - 8, |
| $A^+/(A^+ + NH_4^+)$ | 0,1 - 1, | de préférence 0,3 - 1. |

(b) on maintient ledit mélange réactionnel à une température de chauffage comprise entre 90°C et 300°C, de préférence entre 130°C et 250°C jusqu'à ce que l'on obtienne un composé cristallin.

La présente invention concerne un catalyseur qui contient une zéolithe MFI, préparée selon l'invention de la demande de brevet EP-A-517 891, et dont la formule chimique approchée, après les étapes (a) et (b) décrites ci-dessus de sa synthèse, est la suivante :

$$M^+_{(x+y)}(Si_{(96-(x+y))}Al_xGa_yO_{192})^{(x+y)-},$$

où M représente un cation alcalin et/ou un cation ammonium et/ou un proton. x est un nombre compris entre 0 et 13,7, y est un nombre compris entre 0 et 13,7, (x + y) est un nombre compris entre 0,19 et 13,7.

Dans le but d'utiliser cette zéolithe MFI, préparée selon l'invention de la demande de brevet EP-A-517 891, dans la présente invention, on effectue un ou plusieurs échanges d'ions avec les cations $NH_4^+$ afin de pouvoir obtenir la forme protonée HMFI par calcination sous air à une température supérieure à 350°C, de préférence à 450°C.

La zéolithe MFI peut éventuellement être soumise, après l'étape de calcination de sa forme ammonium, à un traitement de défluoration, partiel ou total, par $NH_4OH$, consistant à traiter ladite zéolithe dans une

solution d'hydroxyde d'ammonium à des températures comprises par exemple entre la température ambiante (15 °C à 25 °C) et 150 °C (traitement sous pression).

Les différentes caractéristiques des zéolithes MFI sont mesurées par les méthodes ci-après :

(a) Les rapports atomiques Si/Al globaux sont déteminés par analyse fluorescence X.

(b) La teneur en fluor est déterminée par dosage à l'aide d'une électrode spécifique.

(c) La cristallinité est évaluée par examen du spectre de diffraction des rayons X.

La zéolithe MFI peut également être soumise à un traitement de désalumination. Il existe diverses méthodes d'obtention de la zéolithe MFI désaluminée ; de manière préférée, la zéolithe MFI forme ammonium ou hydrogène est ainsi soumise à l'un des deux traitements suivants :

1. La zéolithe MFI est soumise à un traitement thermique en présence de vapeur d'eau, qui peut être effectué par la technique du self-steaming (calcination en atmosphère confinée). La température est habituellement comprise entre 300 et 800 °C et, de préférence, entre 400 et 700 °C, pendant un temps habituellement supérieur à 20 minutes. L'atmosphère de calcination contient au moins 1 % et, de préférence, au moins 5 % de vapeur d'eau. Dans le cas du self-steaming, l'atmosphère est constituée essentiellement d'eau et d'ammoniac. Le produit ainsi obtenu peut être soumis à un traitement acide visant à extraire de l'aluminium du solide. Ce traitement peut s'effectuer en plongeant le produit dans un acide minéral ou organique fort de normalité habituellement comprise entre 0,1 et 12 N, à une température usuellement comprise entre 20 et 150 °C et, de préférence, entre 80 et 150 °C, pendant un temps habituellement supérieur à 10 minutes.

2. On procède à un traitement acide, visant à extraire l'aluminium du solide. Ce traitement peut, comme en 1., être effectué en plongeant le solide dans un acide minéral ou organique fort de normalité habituellement comprise entre 0,1 et 15 N, à une température comprise entre 20 et 120 °C, pendant un temps supérieur à 10 minutes.

La présente invention concerne un catalyseur du type galloaluminosilicate caractérisé par la composition suivante exprimée en poids :

(a) 0,01 à 10 % en poids de gallium, de préférence 0,03 à 4 %,

(b) 0,1 à 99,49 % d'une matrice choisie dans le groupe formé par l'alumine, la silice, la magnésie, une argile et toute combinaison d'au moins deux des composés' précités et,

(c) 0,5 à 99,89 % d'une zéolithe, synthétisée en milieu fluorure en l'absence de composés organiques, de formule chimique approchée généralement la suivante :

$$M^{+}_{(x+y)}(Si_{(96-(x+y))}Al_xGa_yO_{192})^{(x+y)-},$$

où M représente un cation alcalin et/ou un cation ammonium et/ou un proton, x est un nombre compris entre 0 et 13,7, y est un nombre compris entre 0 et 13,7, (x + y) est un nombre compris entre 0,19 et 13,7.

La zéolithe MFI, contenue dans le catalyseur selon l'invention, a une teneur en fluor comprise entre 0,01 et 2 % en poids, de préférence 0,02 à 1 % en poids, le fluor étant incorporé lors de la synthèse. De plus, le diagramme de diffraction X de ladite zéolithe est conforme au tableau 1.

La préparation du catalyseur selon l'invention à partir de la zéolithe MFI comprend les opérations décrites ci-après :

La zéolithe, partiellement ou totalement défluorée, peut être soumise telle quelle a un dépôt de gallium puis mise en forme par toutes les techniques connues de l'homme du métier. Cette mise en forme peut également intervenir avant que le dépôt de gallium ait été effectué. Dans ce cas, on procède au dépôt de gallium sur le solide mis en forme.

La zéolithe MFI peut en particulier être mélangée à une matrice généralement amorphe, par exemple à une poudre humide de gel d'alumine. Le mélange est ensuite mis en forme, par exemple par extrusion au travers d'une filière. La teneur en zéolithe du support ainsi obtenu est généralement comprise entre 0,5 et 99,89 % et avantageusement comprise entre 40 et 90 % poids. La teneur en matrice du catalyseur est comprise entre 0,1 et 99,49 % et avantageusement entre 10 et 60 %. La mise en forme peut être réalisée avec d'autres matrices que l'alumine, telles que, par exemple la magnésie, la silice alumine, les argiles naturelles (kaolin, bentonite), et par des techniques telles que l'extrusion, le pastillage, la dragéification ou la coagulation en gouttes (oil drop).

Le dépôt de gallium, sur la zéolithe MFI avant mise en forme ou bien sur le support après mise en forme, est effectué par tout procédé connu de l'homme du métier et permettant le dépôt du métal dans la zéolithe. On peut utiliser la technique d'échange cationique avec compétition où l'agent compétiteur est de préférence le nitrate d'ammonium, ou encore les techniques de dépôt de gallium sur le catalyseur par imprégnation ou par précipitation. Les solutions d'échange ou d'imprégnation du gallium peuvent être

préparées à partir de composés du gallium tels que par exemple l'oxyde de gallium, le nitrate de gallium, le sulfate de gallium, des halogénures de gallium ou l'hydroxyde de gallium. Ces techniques d'échange ionique ou d'imprégnation ou de précipitation peuvent également être utilisées pour déposer le métal directement sur la poudre de zéolithe, avant son mélange éventuel avec une matrice. La teneur en gallium déposée sur le catalyseur à l'issue de ou des étapes d'échange ionique et/ou d'imprégnation ou de précipitation dépend de la teneur en fluor du solide; elle se situe entre 0,01 et 10 % en poids par rapport à l'ensemble du catalyseur, et de préférence entre 0,3 % et 7,0 % en poids.

Le catalyseur, obtenu par les procédures précédentes et pouvant éventuellement subir un traitement de calcination sous air à une température généralement comprise entre 350 °C et 650 °C, est mis en oeuvre pour la réaction d'aromatisation des gaz légers, par exemple du propane et/ou d'un mélange $C_2$-$C_4$ en présence ou non d'oléfines. Cette réaction revêt un intérêt particulier car elle permet de valoriser des résidus d'opérations de raffinage ($C_2$-$C_4$) en des produits à plus haute valeur ajoutée (benzène, toluène, xylènes principalement) tout en contribuant à la production de quantités importantes d'hydrogène indispensable pour les procédés d'hydrotraitement par exemple.

La charge comprenant au moins un produit choisi dans le groupe formé par l'éthane, le propane, le butane, le n-pentane, l'isopentane, les hexanes et les heptanes, en présence ou non d'oléfines, est mise en contact avec le catalyseur de la présente invention à une température comprise entre 400 et 700 ° C, et plus particulièrement entre 480 et 600 ° C dans le cas où la charge comprend un produit choisi dans le groupe formé par l'éthane, le propane et le butane et plus particulièrement entre 420 et 540 ° C dans le cas où la charge comprend un produit choisi dans le groupe formé par le n-pentane, l'isopentane, les hexanes et les heptanes.

Les exemples qui suivent précisent l'invention sans toutefois en limiter la portée.

Tous les catalyseurs utilisés dans les exemples suivants renferment 20 % d'une matrice et 80 % de zéolithe.

Selon une technique particulière, l'aromatisation peut être effectuée dans au moins un réacteur pouvant être mis hors circuit pour régénérer le catalyseur qu il contient ("swing reactor" en langue anglaise). Il convient que le catalyseur soit utilisé sous forme d'extrudés.

En utilisant cette dernière technique, on a constaté des sélectivités en aromatiques très stables dans des périodes de marche de l'ordre de 500 heures et plus selon les conditions opératoires utilisées.

EXEMPLE 1 : Synthèse de la zéolithe MFI-1 selon l'invention.

Dans un autoclave de 0,5 litre en monel (alliage de cuivre et de nickel), on dissout 50,4 g de NaF (1,2 mole) à température ambiante dans 270 g d'eau (15 moles) ; à cette solution sont alors ajoutés sous agitation et successivement 74,45 g de "tixolex 28" échangé par $NH_4$ de rapport Si/Al = 7,45 et 0,75 g de cristaux de zéolithe MFI synthétisée en milieu fluorure et non calcinée (soit environ 1,4 % en poids de la silice engagée).

La composition du mélange réactionnel en termes de rapport molaire est la suivante :

$$Si/Al = 7{,}45;\ F^-/Si = 1{,}36;\ H_2O/Si = 17;\ Na^+/Si = 1{,}36$$

Le pH du mélange réactionnel est d'environ 8.

Le mélange est porté à la température de 200 °C et est maintenu à cette température sous agitation lente pendant 44 heures.

Après cette synthèse, le solide est recueilli par filtration, lavé à l'eau distillée, séché à l'étuve à 80 °C pendant 24 heures.

Le produit obtenu est cristallisé sous forme de zéolithe MFI, comme le montre le diagramme de diffraction des rayons X de sa forme ammonium (obtenue par échanges ioniques avec des cations ammonium) qui est caractéristique de la zéolithe MFI selon l'invention décrite dans la demande de brevet français n° 90/16529. Son rapport molaire $SiO_2/Al_2O_3$, déterminé par analyse chimique, est de 15,1 et le volume de sa maille élémentaire est de 5,410 $nm^3$. La teneur pondérale en fluor de sa forme ammonium est de 1,0 %. On désigne ledit solide par "zéolithe MFI-1".

EXEMPLE 2 : Catalyseur A utilisé conformément à l'invention.

La zéolithe MFI-1 de l'exemple 1 est soumise à deux échanges ioniques successifs (avec filtration et lavage intermédiaires) dans les conditions suivantes :

- $[NH_4NO_3]$ = 4 N,

- V/P = 5cm$^3$/g,
- T = 100 °C.

On fait subir à la zéolithe MFI-1 de l'exemple 1 un traitement d'une durée de 4 heures avec une solution d'ammoniaque de concentration 0,2 N à la température de 100 °C, le produit est ensuite filtré et lavé à l'eau distillée, puis séché à l'étuve à 150 °C.

Après traitement, on obtient un solide dont la cristallinité et le rapport Si/Al ne sont pas altérés mais dont la teneur en fluor est de 0,05 % poids.

La zéolithe MFI-1 est alors soumise à un traitement de désalumination de façon à ajuster son rapport molaire $SiO_2/Al_2O_3$ à une valeur proche de 50. Pour cela, on effectue deux traitements successifs en milieu acide selon les conditions suivantes :

- [$HNO_3$] = 3 N,
- V/P = 5cm$^3$/g,
- T = 100 °C.

La zéolithe ainsi obtenue a un rapport molaire $SiO_2/Al_2O_3$ égal à 53.

Le solide MFI-1 de l'exemple 1 ainsi traité, est mis en forme par extrusion avec un liant ou matrice de type aluminique à raison de 80 % en poids de zéolithe et 20 % en poids de liant.

Le catalyseur A est préparé de la manière suivante : une alumine pseudo boehmite fournie par la société CONDEA est peptisée par ajout d'acide nitrique puis malaxée.

Le catalyseur A est obtenu par mélange de cette pseudo boehmite avec la zéolithe MFI-1 ayant subi la procédure de défluoration partielle décrite ci-dessus.

Cette zéolithe est introduite à raison de 80 g de zéolithe pour 20 g de liant puis malaxée; la pâte obtenue, après ajustement de sa consistance par ajout de petites quantités d'eau, est forcée au travers d'une filière de diamètre 1,4 mm, puis séchée sous flux d'air à 120 °C et calcinée à 550 °C pendant une heure.

Le gallium est déposé sur les extrudés par échange ionique. La solution d'échange est préparée à partir de nitrate de gallium $Ga(NO_3)_3$ 0,15 N. Le pH de la solution de gallium est ajustée à 2,1 avec de l'ammoniaque.

La teneur en gallium atteinte après trois échanges était de 3,10 % en poids par rapport aux extrudés.

EXEMPLE 3

A titre de comparaison, on prépare un catalyseur B en utilisant une zéolithe MFI synthétisée de façon classique en milieu alcalin, à savoir de CBV5020 commercialisée par la société CONTEKA. Le rapport atomique $SiO_2/Al_2O_3$ de cette zéolithe est égal à 56, sa teneur en $Na_2O$ de 200 ppm et sa surface spécifique de 410 m$^2$/g.

Cette zéolithe est ensuite mise en forme par extrusion avec un liant aluminique à raison de 80 % en poids de zéolithe et 20 % en poids de liant, selon la technique opératoire décrite dans l'exemple 2. On introduit alors du gallium (3,10 % en poids) suivant la procédure décrite dans ce même exemple 2.

EXEMPLE 4

Les catalyseurs A et B ont été testés en aromatisation du propane.

Les conditions opératoires étaient les suivantes :

- Température : 550 °C,
- Pression : atmosphérique,
- Débit horaire de charge liquide égal à 2 fois le poids du catalyseur.

Les résultats obtenus sont présentés sur le tableau 2.

TABLEAU 2

| Catalyseur | A | B |
|---|---|---|
| Sélectivités massiques en produits, hors charge (% poids) | | |
| Hydrogène | 6,9 | 6,2 |
| $C_1 + C_2$ | 20,2 | 21,3 |
| Paraffines | 1,5 | 1,8 |
| Oléfines | 9,2 | 10,4 |
| Benzène + Toluène + Xylènes + Ethylbenzène | 58,8 | 55,1 |
| Aromatiques $C_9^+$ | 3.4 | 5,2 |
| Taux de conversion du propane | 73,3% | 73.5% |

On peut constater la supériorité des catalyseurs utilisés selon l'invention, à base de zéolithe de type MFI synthétisée en milieu fluoré en l'absence de composés organiques. En effet, si l'activité des deux catalyseurs A et B est pratiquement identique, la sélectivité en aromatiques, et surtout en benzène, toluène et xylènes, du catalyseur A est nettement meilleure.

EXEMPLE 5

Les catalyseurs A et B ont été utilisés pour la réaction d'aromatisation d'une coupe paraffinique $C_5$-$C_6$ dont la composition pondérale était celle indiquée dans le tableau 3:

TABLEAU 3

| | Paraffines | Naphtènes | Aromatiques | Totaux |
|---|---|---|---|---|
| $C_4$ | 0,06 | - | - | 0,06 |
| $C_5$ | 89,71 | 3,76 | - | 93,47 |
| $C_6$ | 5,33 | 0,86 | 0,06 | 6,25 |
| $C_7$ | 0,11 | - | 0,05 | 0,16 |
| $C_8$ | - | - | 0,04 | 0,04 |
| $C_9$ | 0,01 | - | - | 0,01 |
| Total | 95,22 | 4,62 | 0,15 | 99,99 |

Les conditions opératoires étaient les suivantes :

- Température : 480 °C,
- Pression : 2,5 bars relatifs,
- Débit horaire de charge liquide égal à 2 fois le poids du catalyseur.

A la sortie du réacteur, les sélectivités massiques en produits (hors charge) étaient celles indiquées dans le tableau 4 :

TABLEAU 4

| Catalyseur | A | B |
|---|---|---|
| Hydrogène | 1,7 | 1,6 |
| Méthane | 6.1 | 6.3 |
| Composés paraffiniques et oléfiniques à moins de 4 atomes de carbone | 41,3 | 44.5 |
| Composés paraffiniques et oléfiniques à 4 et plus de 4 atomes de carbone | 10,2 | 9.0 |
| Benzène + Toluène + Xylènes + Ethylbenzène | 33.6 | 32,1 |
| Aromatiques $C_9^+$ | 7,1 | 6,5 |
| Taux de conversion de la coupe paraffinique ($C_5$-$C_6$) | 96.2% | 95,0% |

On peut constater la supériorité des catalyseurs utilisés selon l'invention, à base de zéolithe de type MFI synthétisée en milieu fluoré en l'absence de composés organiques. En effet, l'activité du catalyseur A selon l'invention ainsi que la sélectivité en aromatiques, et surtout en benzène, toluène et xylènes, du catalyseur A selon l'invention sont nettement meilleures.

**Revendications**

**1.** Utilisation pour l'aromatisation d'une coupe légère comprenant des hydrocarbures dont le nombre d'atomes de carbone par molécule est compris entre 2 et 7 d'un catalyseur renfermant en poids :

(a) 0,01 à 10 % de gallium,

(b) 0,1 à 99,49 % d'une matrice choisie dans le groupe formé par l'alumine, la silice, la magnésie, une argile et toute combinaison d'au moins deux des composés précités,

(c) 0,5 à 99,89 % d'une zéolithe MFI, synthétisée en milieu fluorure en l'absence de composés organiques, de formule générale approchée suivante :

$$M^+_{(x+y)}(Si_{(96-(x+y))}Al_xGa_yO_{192})^{(x+y)-},$$

où M représente un élément choisi dans le groupe formé par les cations alcalins, $NH_4^+$ et $H^+$, x est un nombre compris entre 0 et 13,7, y est un nombre compris entre 0 et 13,7, (x + y) est un nombre compris entre 0,19 et 13,7, ladite zéolithe ayant une teneur en fluor comprise entre 0,01 et 2 % en poids, et un diagramme de diffraction des rayons X de sa forme ammonium représenté dans le tableau suivant :

| $D_{hkl}(10^{-10}m)$ | $I/I_o$ |
|---|---|
| 11,14 | 66 |
| 9,99 | 43 |
| 9,72 | 18 |
| 7,44 | 7 |
| 6,71 | 11 |
| 6,37 | 11 |
| 5,99 | 17 |
| 5,70 | 13 |
| 5,58 | 17 |
| 5,38 | 6 |
| 5,14 | 6 |
| 4,98 | 11 |
| 4,62 | 9 |
| 4,37 | 16 |
| 4,27 | 13 |
| 4,10 | 11 |
| 4,01 | 14 |
| 3,86 | 100 |
| 3,82 | 94 |
| 3,75 | 62 |
| 3,73 | 66 |
| 3,65 | 33 |
| 3,60 | 10 |
| 3,49 | 13 |
| 3,45 | 14 |
| 3,36 | 13 |
| 3,32 | 17 |
| 3,05 | 16 |
| 2,98 | 21 |
| 2,95 | 10 |

**2.** Utilisation selon la revendication 1 d'un catalyseur renfermant 0,03 à 4 % de gallium en poids.

**3.** Utilisation selon l'une des revendications 1 et 2 d'un catalyseur dans lequel ladite zéolithe a une teneur en fluor comprise entre 0,02 et 1 % en poids.

**4.** Utilisation selon l'une des revendications 1 à 3 d'un catalyseur dans lequel ladite matrice est une alumine.

**Claims**

**1.** The use of a catalyst for the aromatisation of a light cut comprising hydrocarbons containing 2 to 7 carbon atoms per molecule, the catalyst including, by weight:

(a) 0.01% to 10% of gallium,

(b) 0.1% to 99. 49% of a matrix selected from the group formed by alumina, silica, magnesia, a clay and any combination of at least two of these compounds,

(c) 0.5% to 99.89% of a MFI zeolite, synthesized in a fluorinated medium in the absence of organic compounds, with the following approximate general formula:

$M^{+}_{(x+y)}(Si_{(96-(x+y))}Al_xGa_yO_{192})^{(x+y)-}$,

where M represents an element selected from the group formed by alkali cations, $NH_4^+$ and $H^+$, x is a number between 0 and 13.7, y is a number between 0 and 13.7, and (x + y) is a number between 0.19 and 13.7, said zeolite having a fluorine content of between 0.01% and 2% by weight, and an X ray diffraction pattern for its ammonium form as shown in the following table:

| $D_{hkl}(10^{-10} m)$ | $I/I_o$ |
|---|---|
| 11,14 | 66 |
| 9,99 | 43 |
| 9,72 | 18 |
| 7,44 | 7 |
| 6,71 | 11 |
| 6,37 | 11 |
| 5,99 | 17 |
| 5,70 | 13 |
| 5,58 | 17 |
| 5,38 | 6 |
| 5,14 | 6 |
| 4,98 | 11 |
| 4,62 | 9 |
| 4,37 | 16 |
| 4,27 | 13 |
| 4,10 | 11 |
| 4,01 | 14 |
| 3,86 | 100 |
| 3,82 | 94 |
| 3,75 | 62 |
| 3,73 | 66 |
| 3,65 | 33 |
| 3,60 | 10 |
| 3,49 | 13 |
| 3,45 | 14 |
| 3,36 | 13 |
| 3,32 | 17 |
| 3,05 | 16 |
| 2,98 | 21 |
| 2,95 | 10 |

**2.** Use according to claim 1 of a catalyst containing 0.03% to 4% by weight of gallium.

**3.** Use according to claim 1 or claim 2 of a catalyst in which said zeolite has a fluorine content of between 0.02% and 1% by weight.

**4.** Use according to any one of claims 1 to 3 of a catalyst in which said matrix is an alumina.

**Patentansprüche**

**1.** Verwendung eines Katalysators, welcher bezogen auf Gewicht,

a) 0,01 bis 10 % Gallium,

b) 0,1 bis 99,49 % einer Matrix, die aus der Gruppe ausgewählt ist, die durch Aluminiumoxid, Siliciumdioxid, Magnesiumoxid, einem Ton und allen Kombinationen von wenigstens zwei der vorher erwähnten Verbindungen gebildet ist,

c) 0,5 bis 99,89 % eines in Fluoridmilieu in Abwesenheit von organischen Verbindungen synthetisierten MFI-Zeoliths, der folgende allgemeine Überschlagsformel

$$M^{+}_{(x+y)}\ [Si_{(96-(x+y))}Al_xGa_yO_{192}]^{(x+y)-},$$

umfaßt,

worin M ein Element bedeutet, das aus der Gruppe ausgewählt ist, die durch die Alkalikationen, $NH_4^+$ und $H^+$ gebildet ist, x eine Zahl ist, die zwischen 0 und 13,7 enthalten ist, y eine Zahl ist, die zwischen 0 und 13,7 enthalten ist, (x + y) eine Zahl ist, die zwischen 0,19 und 13,7 enthalten ist, wobei der Zeolith einen Gehalt an Fluor, der zwischen 0,01 und 2 Gew.-% enthalten ist und ein

Röntgenbeugungsdiagramm seiner Ammoniumform, das in der folgenden Tabelle dargestellt ist:

| $D_{hkl}(10^{-10} m)$ | I/Io |
|---|---|
| 11,14 | 66 |
| 9,99 | 43 |
| 9,72 | 18 |
| 7,44 | 7 |
| 6,71 | 11 |
| 6,37 | 11 |
| 5,99 | 17 |
| 5,70 | 13 |
| 5,58 | 17 |
| 5,38 | 6 |
| 5,14 | 6 |
| 4,98 | 11 |
| 4,62 | 9 |
| 4,37 | 16 |
| 4,27 | 13 |
| 4,10 | 11 |
| 4,01 | 14 |
| 3,86 | 100 |
| 3,82 | 94 |
| 3,75 | 62 |
| 3,73 | 66 |
| 3,65 | 33 |
| 3,60 | 10 |
| 3,49 | 13 |
| 3,45 | 14 |
| 3,36 | 13 |
| 3,32 | 17 |
| 3,05 | 16 |
| 2,98 | 21 |
| 2,95 | 10 |

besitzt, zur Aromatisierung eines leichten Schnittes, der Kohlenwasserstoffe umfaßt, deren Zahl von Kohlenstoffatomen pro Molekül zwischen 2 und 7 liegt.

**2.** Verwendung nach Anspruch 1 eines Katalysators, der 0,03 bis 4 Gew.-% Gallium umfaßt.

**3.** Verwendung nach einem der Ansprüche 1 und 2 eines Katalysators, worin der Zeolith einen Gehalt an Fluor aufweist, der zwischen 0,02 und 1 Gew.-% liegt.

**4.** Verwendung nach einem der Ansprüche 1 bis 3 eines Katalysators, worin die Matrix ein Aluminiumoxid ist.